(19) Europäisches Patentamt
European Patent Office
Office européen des brevets

(11) EP 0 882 700 A2

(12) # EUROPEAN PATENT APPLICATION

(43) Date of publication:
09.12.1998 Bulletin 1998/50

(51) Int. Cl.[6]: C07C 211/26

(21) Application number: 97115338.2

(22) Date of filing: 04.09.1997

(84) Designated Contracting States:
AT BE CH DE DK ES FI FR GB GR IE IT LI LU MC NL PT SE
Designated Extension States:
AL LT LV RO SI

(30) Priority: 03.06.1997 IT BO970331

(71) Applicant:
ALFA CHEMICALS ITALIANA S.p.A.
24122 Bergamo (IT)

(72) Inventors:
- Cannata, Vincenzo
Nuovo di Pontecchio Marconi, Bologna (IT)
- Bianchi, Stefano
22100 Como (IT)
- Spreafico, Angelo
22053 Lecco (IT)
- Maspes, Barbara
20133 Milano (IT)

(74) Representative:
Kraus, Walter, Dr. et al
Patentanwälte Kraus, Weisert & Partner
Thomas-Wimmer-Ring 15
80539 München (DE)

## (54) Process for the production of the dexfenfluramine hydrochloride

(57) Process for the production of the anorexic drug dexfenfluramine hydrochloride (INN) which comprises the ethylation of the (S),(S)-N-(1-phenylethyl)-α-methyl-3-(trifluoromethyl)benzeneethanamine or of a salt thereof with an organic or inorganic acid, the debenzylation of the resulting intermediate (S),(S)-N-ethyl-N-(1-phenylethyl)-α-methyl-3-(trifluoromethyl)benzeneethanamine or of a salt thereof with an organic or inorganic salt by catalytic hydrogenation and, optionally, the salification with hydrochloric acid.

EP 0 882 700 A2

## Description

### BACKGROUND OF THE INVENTION

The dexfenfluramine hydrochloride (INN), dextrorotatory isomer of the fenfluramine hydrochloride (INN), is a well-known anorexic drug described in US Patent 3,198,833 wherein the separation of the two optical isomers of the fenfluramine to get the dextrorotatory isomer much more active than the levorotatory isomer is reported.

This separation bases itself on a classic method of optical resolution through the formation of diastereoisomer salts first with the (+)-dibenzoyltartaric acid and then with the (+)-camphoric acid.

The yield in dexfenfluramine is very low, about 11% calculated over the racemate, and the process is very troubled as it requires the crystallization of two different diastereoisomer salts.

Stereospecific syntheses have been tried in order to get only the dexfenfluramine to avoid the low yields and the high costs proper to the separation of the optical isomers through the crystallization of the diastereoisomer salts with optically active acids.

European Patent 0,301,925 describes the enantiospecific production of the dexfenfluramine by starting from the (S)-2-amino-1-propanol through a series of stereospecific condensations and reductions.

European Patent 0,441,160 in its turn describes the enantiospecific production of the dexfenfluramine starting from (S)-1-(3-trifluoromethyl)phenyl-2-propanol obtained by inversion of the (R)-1-(3-trifluoromethyl)phenyl-2-propanol obtained in its turn by enantioselective enzymatic reduction of the corresponding ketone.

The present invention refers to a new process for the production of the dexfenfluramine hydrochloride starting from new intermediates described in the Italian Patent application BO97A000252, i.e. the (S),(S)-N-(1-phenylethyl)-α-methyl-3-(trifluoromethyl)benzeneethanamine and the salts thereof with organic or inorganic acids.

This process envisages a first step which is made up of the ethylation of (S),(S)-N-(1-phenylethyl)-α-methyl-3-(trifluoromethyl) benzeneethanamine or of a salt thereof with an organic or inorganic acid followed by a reductive debenzylation of the resulting new intermediate (S),(S)-N-ethyl-N-(1-phenylethyl)-α-methyl-3-(trifluoromethyl)benzeneethanamine or of a salt thereof with an organic or inorganic acid and, optionally, by the salification with hydrochloric acid.

### DESCRIPTION OF THE INVENTION

Process for the production of the dexfenfluramine hydrochloride of formula

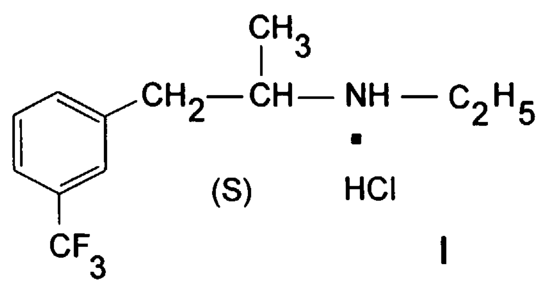

I

which comprises:

*a)* reacting the (S),(S)-N-(1-phenylethyl)-α-methyl-3-(trifluoromethyl)benzeneethanamine of formula

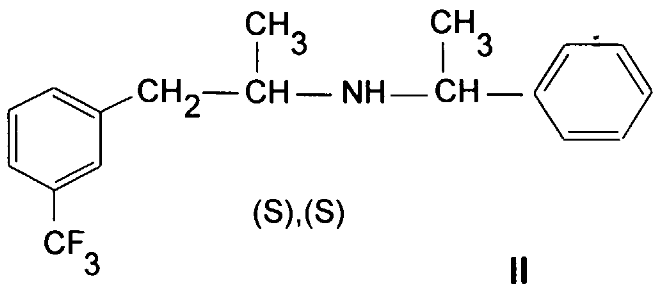

(S),(S)

II

or a salt thereof with an organic or inorganic acid with an ethylating agent in order to obtain the (S),(S)-N-ethyl-N-(1-phenylethyl)-α-methyl-3-(trifluoromethyl)benzeneethanamine of formula

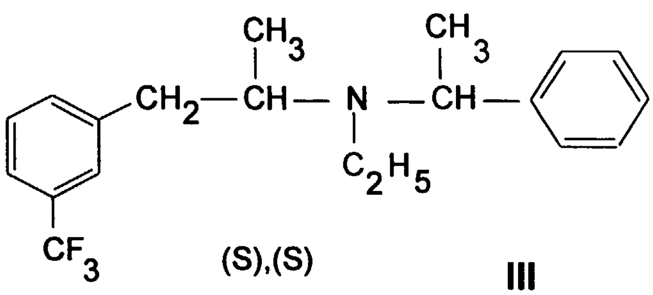

(S),(S)

III

or a salt thereof with an organic or inorganic acid;

*b)* debenzylating the compound of formula III or a salt thereof with an organic or inorganic acid by hydrogenation in the presence of a catalyst;

*c)* carrying out, optionally, the salification with hydrochloric acid.

The intermediate of formula III and the salts thereof with organic or inorganic acids are new products and are claimed as such.

The salt preferred in carrying out the invention is the hydrochloride.

The reaction of ethylation is carried out by reacting a molar equivalent of (S),(S)-N-(1-phenylethyl)-α-methyl-3-(trifluoromethyl) benzeneethanamine or of a salt thereof with an organic or inorganic acid with from 1 to 3 molar equivalents of an alkylating agent in the presence of from 1 to 3 molar equivalents of a non-quaternizable tertiary amine or of an alkaline carbonate for a period of time between 4 and 80 hours at a temperature between 20°C and 150°C. In a preferred aspect of the invention the alkylating agent is selected from ethyl bromide, ethyl iodide, diethyl sulfate, ethyl metanesulfonate and ethyl p-toluenesulfonate, the polar solvent is selected from N,N-dimethylformamide N-methylpyrrolidone, N,N-dimethylacetamide and triethylphosphate, the tertiary amine is selected from N,N-diisopropylethylamine, N,N-diisopropylpropylamine N,N-diisobutylethylamine, N,N-diisopropylallylamine and N,N-dicyclohexylethylamine and the alkaline carbonate is selected from sodium and potassium carbonates.

The intermediate (S),(S)-N-ethyl-N-(1-phenylethyl)-α-methyl-3-(trifluoromethyl)benzeneethanamine can be used as such for the subsequent reaction of debenzylation to dexfenfluramine or can be transformed in a crystalline salt with an organic or inorganic acid.

In a preferred aspect of the invention it is dissolved in an organic solvent immiscible with water, preferably an aromatic hydrocarbon like the toluene, and the hydrochloride in form of crystalline precipitate is obtained by adding an aqueous concentrated solution of hydrochloric acid.

The reaction of debenzylation is carried out by submitting the intermediate (S),(S)-N-ethyl-N-(1-phenylethyl)-α-methyl-3-(trifluoromethyl)benzeneethanamine or a salt thereof with an organic or inorganic acid, preferably the hydrochloride to catalytic hydrogenation in a polar solvent or mixture of polar and non-polar solvents under from 1 to 20 hydrogen atmospheres, at a temperature between 20°C and 100°C for a period of time between 1 and 8 hours.

The polar solvent preferred in carrying out the invention is selected from the alcohols containing from 1 to 6 carbon atoms while palladium at 5% on carbon is the preferred catalyst. At the end of the reaction of debenzylation, the raw product of reaction can be transformed into the pure dexfenfluramine hydrochloride by treating the dexfenfluramine

base, directly obtained in the benzylation of the intermediate of formula III or by treatment with an aqueous solution of an alkaline hydroxide when the debenzylation is carried out on an organic or inorganic salt of the intermediate of formula III, with gaseous hydrochloric acid in a solvent or mixture of solvents.

The dexfenfluramine base is dissolved in a solvent or in a mixture of solvents selected from the aliphatic ketones containing from 3 to 8 carbon atoms, the alcohols containing from 1 to 6 carbon atoms, the acetates of the alcohols containing from 1 to 6 carbon atoms and the alkyl ethers containing from 4 to 8 carbon atoms and anhydrous gaseous hydrochloric acid is insufflated into the solution until the colour change of the congo red indicator.

When the raw dexfenfluramine hydrochloride is directly obtained from the reaction of debenzylation it can be also directly purified without an advance transformation into dexfenfluramine base.

In this case the raw dexfenfluramine hydrochloride, obtained by evaporating the solvent used in the reaction of debenzylation is crystallised by hot solubilization in a solvent or mixture of solvents previously described for the salification with gaseous hydrochloric acid. The aliphatic ketones containing from 3 to 8 carbon atoms are preferred.

The below reported examples are a further illustration of the invention and do not have to be taken as an its limitation.

## EXAMPLE 1

**(S),(S)-N-ethyl-N-(1-phenylethyl)-α-methyl-3-(trifluoromethyl) benzeneethanamine hydrochloride**

A mixture made by 100 g (0.326 moles) of (S),(S)-N-(1-phenylethyl)-α-methyl-3-(trifluoromethyl)benzeneethanamine, 71 g (0.456 moles) of ethyl iodide and 58.9 g (0.456 moles) of N,N-diisopropylethylamine in 200 ml of N,N-dimethylformamide is heated to 60°C under stirring for 60 hours. Then the reaction mixture is concentrated to 90°C under vacuum, at the pressure of 10 mm Hg, and the raw product so obtained is added with 200 ml of water and 200 ml of toluene and the mixture is alkalinized to pH 12 by means of a 30% (w/w) aqueous solution of sodium hydroxide. The two layers are then separated, the aqueous layer is discarded while the organic layer is added with 200 ml of water and acidified with a 37% (w/w) aqueous solution of hydrochloric acid. The mixture is cooled in 30 minutes to 5°C under stirring and the precipitated solid is filtered, washed first with water and then with toluene and dried under vacuum at 65°C for 12 hours.

116 Grams of product with m.p. = 196°-198° C and $[\alpha]_D^{25}$ = +49.6° (c = 2% in absolute ethanol) are obtained with a yield equal to 95.5%.

## EXAMPLE 2

**(S),(S)-N-ethyl-N-(1-phenylethyl)-α-methyl-3-(trifluoromethyl) benzeneethanamine hydrochloride**

A mixture made by 461 g (1.505 moles) of (S),(S)-N-(1-phenylethyl)-α-methyl-3-(trifluoromethyl)benzeneethanamine 254 g (2.331 moles) of ethyl bromide, 290 g (2.235 moles) of N,N-diisopropylethylamine and 600 ml of N,N-dimethylformamide is put into an autoclave. The autoclave is closed and the reaction mixture is kept at 100°C for 40 hours under stirring. The reaction mixture is then cooled to 50°C and added with 500 ml of water and 225 ml of a 30% (w/w) aqueous solution of sodium hydroxide. The mixture is added with 500 ml of toluene after 30 minutes under stirring and kept under stirring for other 30 minutes. The phases are then separated, the aqueous layer is eliminated while the organic layer is filtered through a bed of decolorizing earths, added with 200 ml of water and then, in 30 minutes, of 230 ml of a 37% (w/w) aqueous solution of hydrochloric acid. The crystallization of the product is started with 2 g of crystals of pure product and another 37% (w/w) aqueous solution of hydrochloric acid is added until the pH of the mixture is brought to the constant value of 1.5.

The suspension is then cooled to 5°C and filtered and the crystalline solid is washed with water and dried under vacuum at 70°C for 12 hours.

502 Grams of pure product are obtained with a yield equal to 90%.

## EXAMPLE 3

**(S),(S)-N-ethyl-N-(1-phenylethyl)-α-methyl-3-(trifluoromethyl) benzeneethanamine**

50 Grams of (S),(S)-N-ethyl-N-(1-phenylethyl)-α-methyl-3-(trifluoromethyl)benzeneethanamine hydrochloride and 17 ml of a 30% (w/w) aqueous solution of sodium hydroxide are added, under stirring, to a mixture made by 200 ml of water and 200 ml of methylene chloride.

The phases are then separated, the aqueous layer is discarded while the organic layer is twice washed with 100 ml of water and then evaporated under vacuum in order to completely eliminate the solvent.

42.7 Grams of (S),(S)-N-ethyl-N-(1-phenylethyl)-α-methyl-3-(trifluoromethyl)benzeneethanamine are obtained in the form of a colourless oil having $[\alpha]^{25}_{D}$ = +54.1° (c = 2% in absolute ethanol) with a yield equal to 95%.

**EXAMPLE 4**

**(S)-N-ethyl-α-methyl-3-(trifluoromethyl)benzeneethanamine hydrochloride (dexfenfluramine hydrochloride)**

49.5 Grams (0.133 moles) of (S),(S)-N-ethyl-N-(1-phenylethyl)-α-methyl-3-(trifluoromethyl)benzeneethanamine hydrochloride 250 ml of methanol and 1.0 g of palladium on carbon wet at 50% are put into a hydrogenator.

An atmosphere of hydrogen at the constant pressure of 5 atmospheres at the temperature of 75°C for a period of time of 2 hours is kept into the hydrogenator. The reaction mixture is then filtered and the solvent is evaporated under vacuum obtaining a solid which is treated with a mixture made by 100 ml of water and 60 ml of toluene. The organic layer is discarded while the aqueous layer is alkalinized to pH 11.4 with a 30% (w/w) aqueous solution of sodium hydroxide. The oily suspension so obtained is extracted once with 100 ml of methylene chloride and twice with 30 ml of methylene chloride and the organic extracts are collected, washed twice with 20 ml of water and evaporated under vacuum. The clear colourless oil so obtained is dissolved at room temperature into 330 ml of methylethylketone and gaseous anhydrous hydrochloric acid is bubbled into the solution until the colour change to the congo red. The so obtained suspension is cooled under stirring at 5°C and filtered.

The obtained solid is washed with 30 ml of methylethylketone and dried under vacuum for 12 hours at 60°C to give 30 g of pure dexfenfluramine hydrochloride having m.p. = 160.2°C with a yield equal to 84.4%.

The corresponding free base has $[\alpha]^{25}_{D}$ = +9.25° (c = 8% in absolute ethanol).

**EXAMPLE 5**

**(S)-N-ethyl-α-methyl-3-(trifluoromethyl)benzeneethanamine hydrochloride (dexfenfluramine hydrochloride)**

400 Grams (1.075 moles) of (S),(S)-N-ethyl-N-(1-phenylethyl)-α-methyl-3-(trifluoromethyl)benzeneethanamine hydrochloride 400 ml of methanol and 2.0 g of 5% palladium on carbon wet at 50% are put in a hydrogenator. An atmosphere of hydrogen at the constant pressure of 5 atmospheres at the temperature of 50°C for a period of time of 4 hours is kept into the hydrogenator. The reaction mixture is then cooled to 20°C and filtered in order to eliminate the catalyst and the obtained solution is concentrated to dryness obtaining a crystalline solid. This solid is dissolved with 500 ml of boiling methylisobutylketone, then 300 ml of solvent are distilled off and 300 ml of methylisobutylketone are added.

The pure product crystallizes by cooling to 0°C for two hours, then it is filtered, washed on the filter with 200 ml of methylisobutylketone and dried under vacuum at 60°C for 12 hours.

260 Grams of pure dexfenfluramine hydrochloride are obtained with a yield equal to 90%.

The corresponding free base has $[\alpha]^{25}_{D}$ = +9.4° (c = 8% in absolute ethanol).

**Claims**

1. Process for the production of the dexfenfluramine hydrochloride of formula

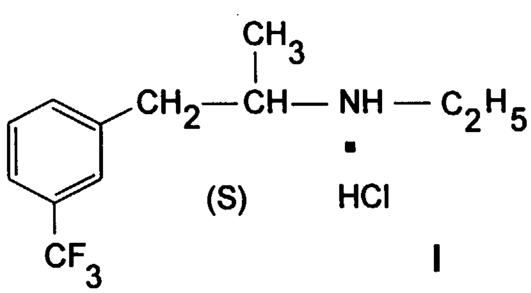

I

which comprises:

*A)* reacting the (S),(S)-N-(1-phenylethyl)-α-methyl-3-(trifluoromethyl)benzeneethanamine of formula

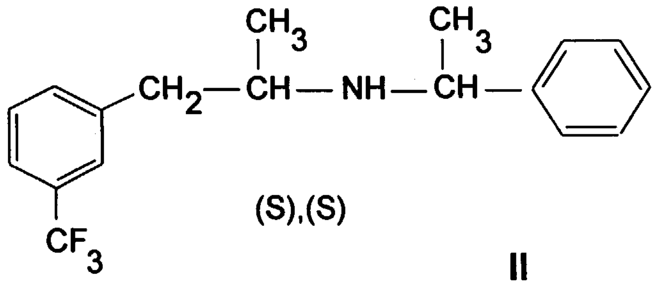

(S),(S)

II

or a salt thereof with an organic or inorganic salt with an ethylating agent in order to get the (S),(S)-N-ethyl-N-(1-phenylethyl)-α-methyl-3-(trifluoromethyl)benzeneethanamine of formula

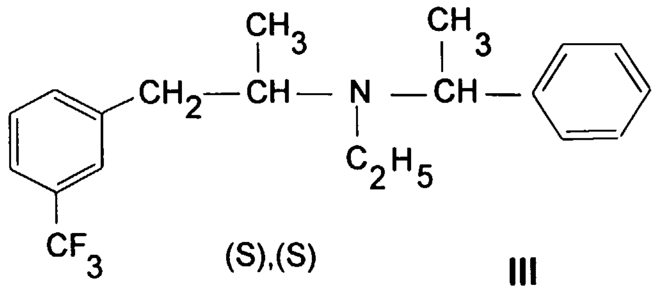

(S),(S)

III

or a salt thereof with an organic or inorganic acid;

B) debenzylating the compound of formula III or a salt thereof with an organic or inorganic acid by hydrogenation in the presence of a catalyst;

C) carrying out, optionally, the salification with hydrochloric acid.

2. Process according to claim 1 characterized in that the reaction of ethylation is carried out in a polar solvent in the presence of from 1 to 3 molar equivalents of a non-quaternarizable tertiary amine or of an alkaline carbonate with respect to the (S),(S)-N-(1-phenylethyl)-α-methyl-3-(trifluoromethyl)benzeneethanamine or to a salt thereof with an organic or inorganic acid at a temperature between 20°C and 150°C, for a period of time between 4 and 80 hours using from 1 to 3 molar equivalents of an alkylating agent selected from ethyl bromide, ethyl iodide, diethyl sulfate, ethyl methanesulfonate or ethyl p-toluene-sulfonate, that the debenzylation is carried out in a hydrogenator in a solvent made by a polar solvent or a mixture of polar and non-polar solvents under from 1 to 20 hydrogen atmospheres at a temperature between 20°C and 100°C for a period of time between 1 and 8 hours using as catalyst 5% palladium on carbon an that the salification occurs by treating the dexfenfluramine dissolved in a solvent or mixture of solvents with gaseous hydrochloric acid.

3. Process according to claim 2 characterized in that the non-quaternarizable tertiary amine is selected from N,N-diisopropylethylamine, N,N-diisobutylethylamine N,N-diisopropylpropylamine, N,N-diisopropylallylamine and N,N-dicyclohexylethylamine and the alkaline carbonate is selected from the sodium and potassium carbonates, that the polar solvent in which the ethylation is carried out is selected from N,N-dimethylformamide, N-methylpyrrolidone, N,N-dimethylacetamide and triethylphosphate, that the polar solvent in which the debenzylation is carried out is selected from the alcohols containing from 1 to 6 carbon atoms and that the solvent used for the salification of the dexfenfluramine is selected from the alcohols containing from 1 to 6 carbon atoms, the aliphatic ketones containing from 3 to 8 carbon atoms, the acetates of the alcohols containing from 1 to 6 carbon atoms, the alkyl ethers containing from 4 to 8 carbon atoms or mixtures thereof.

4. (S),(S)-N-ethyl-N-(1-phenylethyl)-α-methyl-3-(trifluoromethyl) benzeneethanamine.

5. Salts of the (S),(S)-N-ethyl-N-(1-phenylethyl)-α-methyl-3-(trifluoromethyl)benzeneethanamine with organic or inorganic acids.

6. A salt according to claim 5 characterized in that it is the hydrochloride.